# EUROPEAN PATENT SPECIFICATION

(11) **EP 2 416 731 B1**
(45) Date of publication and mention of the grant of the patent: **03.12.2014**
(21) Application number: 10751217.0
(22) Date of filing: 05.03.2010
(51) Int. Cl.: A61F 2/00, A61L 29/16, A61L 29/06

(54) **MEDICAL DEVICES CONTAINING NITROPRUSSIDE AND ANTIMICROBIAL AGENTS**
MEDIZINGERÄTE MIT NITROPRUSSID UND ANTIMIKROBIELLEN WIRKSTOFFEN
DISPOSITIFS MÉDICAUX CONTENANT DU NITROPRUSSIATE ET DES AGENTS ANTIMICROBIENS

(30) Priority: 11.03.2009 US 401829
(43) Date of publication of application: 15.02.2012
(60) Divisional application: 14157870.8
(73) Proprietor: Teleflex Medical Incorporated, Durham, NC 27709 (US)
(72) Inventor: GUPTA, Nisha, Audubon Pennsylvania 19403 (US); ROSENBLATT, Joel, Pottstown Pennsylvania 19465 (US); STEINKE, Elaine, Morgantown Pennsylvania 19543 (US)
(74) Representative: von Hirschhausen, Helge
(86) International application number: PCT/US2010/026339
(87) International publication number: WO 2010/104760

(56) References cited:
- WO-A1-01/70199
- WO-A1-2006/125262
- WO-A1-2007/127488
- WO-A1-2008/150375
- US-A1- 2006 194 008
- US-A1- 2007 010 702
- US-A1- 2007 129 690
- US-A1- 2007 207 335
- US-A1- 2007 224 235
- US-A1- 2007 248 676
- US-A1- 2008 014 557
- US-B1- 6 656 217

## Description

### FIELD OF INVENTION

The present invention generally relates to medical devices having beneficial biological properties. More particularly, the present invention pertains to medical devices having antithrombogenic and antimicrobial properties and a method of production thereof.

### BACKGROUND OF THE INVENTION

Catheters are presently utilized in a great variety of medical procedures. Typically, these catheters are fabricated from polymers such as polyurethane, silicone etc. It is generally known that central venous catheters widely used in clinical settings develop a fibrin sheath within days of insertion into a patient. Aside from reducing the function of the catheter, catheter-related thrombi may arise. WO 2008/150375 A1 relating to combined fibrinolytic and antimicrobial catheters addresses also the problem of fibrin sheath development.

Studies by L. J. Ignarro, published in Nitric Oxide: Biology and Pathobiology, Academic Press, San Diego (2000), indicate that the non-thrombogenic properties of vascular endothelium are primarily attributed to continuous release of NO (nitric oxide) by these endothelial cells into the lumen of blood vessels at an estimated flux of 0.5 to 1.0 X 10⁻¹⁰ mol cm⁻² min⁻¹. It has been proposed that by mimicking the physiological release of NO, the biocompatibility of catheters may be improved.

NO is a free radical endogenously synthesized in the human body when L- arginine is converted to L-citrulline by a class of enzymes known as nitric oxide synthases. NO regulates a range of crucial biological processes in the cardiovascular, gastrointestinal, genitourinary, respiratory, and central and peripheral nervous systems. The discovery of NO as a potent inhibitor of platelet adhesion and activation, (G.-R. Wang et al (1998) Mechanism of platelet inhibition by nitric oxide: In vivo phosphorylation of thromboxane receptor by cyclic GMP-dependent protein kinase. PNAS 95: 4888-4893) and its identification as both an antimicrobial (S. Carlsson et al (2005) Intravesical nitric oxide delivery for prevention of catheter-associated urinary tract infections. Antimicrobial agents and Chemotherapy 49: 2352-2355; F. C. Fang (1997) Mechanisms of Nitric Oxide-related Antimicrobial Activity. J. Clin. Invest. Volume 99: 2818-2825; and F. C. Fang et al (1997) Perspectives series: host/pathogen interactions. Mechanisms of nitric oxide-related antimicrobial activity. J. Clin. Invest. 99: 2818-2825) and anti-inflammatory agent (R. M. Clancy et al (1998) The role of nitric oxide in inflammation and immunity. Arthritis & Rheumatism 41: 1111-1151 and D. Vernet et al (2002) Effect of nitric oxide on the differentiation of fibroblasts into myofibroblasts in the Peyronie's fibrotic plaque and in its rat model. Nitric Oxide 7: 262-276) have extended NO research to the field of biomaterials.

WO 2006/125262 A1 describes a method for regulating biofilm development by exposing the biofilm to nitric oxide or at least one nitric oxide generating releasing agent.

Various approaches have been utilized to impart NO releasing capabilities at device surfaces including incorporating NO donors such as diazeniumdiolates and nitrosothiols (T. Peters (2006) *World Intellectual Property Organization (WIPO)* WO06100156 A3 and *European Patent* EPO 1704879 A1; J. H. Shin and M. H. Schoenfisch (2006) Improving the biocompatibility of in vivo sensors via nitric oxide release. The Analyst 131 : 609-615; M.H. Schoenfisch, K.A. Mowery, M.V. Rader, N. Baliga, J.A. Wahr, M.E. Meyerhoff (2000) Improving the thromboresistivity of chemical sensors via nitric oxide release: Fabrication and in vivo evaluation of NO-releasing oxygensensing catheters. Anal. Chem. 72: 1 1 19-1126; H.P. Zhang, G.M. Annich, J. Miskulin, K. Osterholzer, S.I. Merz, R.H. Bartlett, M.E. Meyerhoff (2002) Nitric oxide releasing silicone rubbers with improved blood compatibility: preparation, characterization, and in vivo evaluation. Biomaterials 23: 1485-1494; M.C. Frost, S.M. Rudich, H.P. Zhang, M. A. Maraschio, M.E.Meyerhoff (2002) In vivo biocompatibility and analytical performance of intravascular amperometric oxygen sensors prepared with improved nitric oxide-releasing silicone rubber coating. Anal. Chem. 74 (2002) 5942-5947). However, these NO donors may be relatively complicated to synthesize and may in some instances require stringent storage conditions. The use of polymer films doped with small metallic copper particles as the catalytic coatings on the surface of intravascular electrochemical oxygen sensing catheters has also been described (Y. Wu et al (2007) Improving blood compatibility of intravascular oxygen sensors via catalytic decomposition of S-nitrosothiols to generate nitric oxide in situ. Sensors and Actuators .5 121 : 36-46.). Such coatings can generate NO in situ at blood interfaces via a slow corrosion of copper particles to produce copper ions. Another approach used has been coating the device with a polymer coating with dissolved or dispersed organometallic nitrosyl compound such as sodium nitroprusside (SNP), a slow NO donor (Rosen et al (1998) Medical device with a surface adapted for exposure to a blood stream which is coated with a polymer containing a nitrosyl-containing organometallic compound which releases nitric oxide from the coating to mediate platelet aggregation; United States Patent No. 5,797,887 and Herzog, Jr. et al (2003) Medical device coated with a polymer containing a nitric oxide releasing organometallic nitrosyl compound useful for the prevention of platelet aggregation; United States Patent No. 6,656,217.). Sodium nitroprusside when is contact with blood releases NO at physiologically relevant levels.

In addition to catheter-related thrombi, the United States Centers for Disease Control (CDC), estimates there are 200,000 to 400,000 episodes of catheter related blood stream infections (CRBSI) annually. One approach utilized to reduce the incidence of CRBSI, is the use of antimicrobials which have either been incorporated into, or used to coat, catheter polymers. Catheters coated with antiseptics (D. L. Veenstra et al (1999) Efficacy of Antiseptic-Impregnated Central Venous Catheters in Preventing Catheter- Related Bloodstream Infection- a meta-analysis. JAMA 281 :261-267) or antibiotics (I. Raad et al (1997) Central venous catheters coated with minocycline and rifampin for the prevention of catheter-related colonization and bloodstream infections. A randomized, double-blind trial. The Texas Medical Center Catheter Study Group. Ann Intern. Med. 127: 267 -274) have been shown effective in significantly lowering the incidence of CRBSI. However, there is no indication that these or other antimicrobial agents would be compatible with NO or NO donor. To the contrary, due to the free radical nature of NO, it may be expected to inactivate or reduce the activity of antimicrobial agents.

Accordingly, it is desirable to provide an implantable medical device having an antithrombogenic agent and antimicrobial agent combination that is compatible and/or method of fabricating an implantable medical device having a compatible combination of an antithrombogenic agent and antimicrobial agent that is capable of overcoming the disadvantages described herein at least to some extent.

### SUMMARY OF THE INVENTION

The foregoing needs are met, to a great extent, by the present invention, wherein in one respect an implantable medical device having a compatible combination of an antithrombogenic agent and antimicrobial agent and method of fabricating the medical device is provided.

An embodiment of the present invention pertains to a medical device including a catheter fabricated by a method according to claim 1. The medical device includes a surface having nitroprusside and an antimicrobial agent.

Another embodiment of the present invention relates to a medical device including a catheter fabricated according to any of the dependent claims 2 to 10. The medical device includes a structure configured for introduction into a vascular system of a patient. The structure includes a surface having nitroprusside and a chlorhexidine salt according to one of claims 3 to 5 or an antimicrobial dye according to claim 6 or an antibiotic agent according to claim 7 disposed thereupon. The nitroprusside has a concentration sufficient to reduce thrombosis.

Yet another embodiment of the present invention pertains to a method of fabricating a medical catheter. In this method, a base material is impregnated with nitroprusside, the medical catheter is formed from the base material, and the medical catheter is coated with an antimicrobial agent.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

### BRIEF DESCRIPTION OF THE DRAWINGS

FIG. 1 is a graph showing the effect of sodium nitroprusside on platelet aggregation as measured in resistance (ohms) and platelet activation as measured by the amount of adenosine triphosphate released (nM).
FIG. 2 is a graph showing nitric oxide generation from a coating of sodium nitroprusside alone as compared to nitric oxide generation from a coating of the combination of silver and sodium nitroprusside.
FIG. 3 is a graph showing biodurability of a coating of chlorhexidine diacetate, sodium nitroprusside, and silver as measured by the concentration of nitric oxide released over time.
FIG. 4 is a graph showing biodurability of a coating of sodium nitroprusside, and silver over gentian violet extrusions as measured by the concentration of nitric oxide released over time.
FIG. 5 is a graph showing biodurability of a coating of chlorhexidine palmitate, sodium nitroprusside, and gentian violet as measured by the concentration of nitric oxide released over time.
FIG. 6 is a graph showing synergy of sodium nitroprusside with chlorhexidine palmitate, and gentian violet against *Pseudomonas aeruginosa.*

### DETAILED DESCRIPTION

The invention provide methods of fabricating medical devices. In various embodiments, medical devices are coated or impregnated with nitroprusside (hereinafter "NP") which functions as an NO (nitric oxide) donor. In a preferred form, the NP includes sodium nitroprusside (hereinafter "SNP") a sodium salt of NP. These medical devices are implantable catheters, and preferably release nitric oxide over an extended period of time. An unexpected benefit has been discovered that SNP functions synergistically with antimicrobial agents such as chlorhexidine, antimicrobial dyes, and rifampin to provide enhanced antimicrobial protection especially against refractory gram negative pathogens. Particular examples of antimicrobial dyes include gentian violet, methyl violet, brilliant green, methylene blue, and the like. As shown and described herein, SNP, in addition to providing an antithrombogenic effect by releasing NO, surprisingly also has either additive or synergistic effect when present in combination with antiseptic and antibiotic agents against refractory gram negative bacteria such as *Pseudomonas aeruginosa.* The unexpected compatibility of SNP with antiseptic agents, antibiotics, antimicrobial metals, and dyes utilized in fabricating antimicrobial medical devices and the durability of such combinations in physiological environments have not been previously reported.

In addition, it is within the.purview of this and other embodiments of the invention that other suitable agents may be incorporated into the bulk material. Examples of suitable agents includes other antibiotics, antiseptics, chemotherapeutics, antimicrobial peptides, mimetics, antithrombogenics, fibrinolytics, anticoagulants, anti-inflammatory agents, anti-pain agents, antinausea agents, vasodilators, antiproliferatives, antifibrotics, growth factors, cytokines, antibodies, peptides and peptide mimetics, nucleic acids, and/or the like.

Medical devices suitable for use with various embodiments of the invention are catheters. In various embodiments, the SNP, chlorhexidine, rifampin, gentian violet and/or the like may be incorporated in or on all or part of the medical device. In a particular example, the SNP, chlorhexidine, and gentian violet may be applied to or near the tip area of a vascular catheter. In this manner, the bio-active constituents may be localized at or near the portion of the catheter most likely to be in contact with blood and/or blood products.

Forms of chlorhexidine suitable for use with embodiments of the invention include chlorhexidine base, pharmaceutically acceptable chlorhexidine salts such as, for example, diacetate, laurate (dodecanoate), palmitate (hexadecanoate), myristate (tetradecanoate), stearate (octadecanoate) and/or the like. In addition, while particular examples are made of chlorhexidine base, chlorhexidine diacetate, and chlorhexidine dodecanoate, embodiments of the invention are not limited to any one form. Instead, as used herein, the term, 'chlorhexidine' refers to any one or a mixture of chlorhexidine base, pharmaceutically acceptable chlorhexidine salts such as, for example, diacetate, dodecanoate, palmitate, myristate, stearate and/or the like. For example, other suitable chlorhexidine salts are to be found in U.S. Patent 6,706,024, entitled Triclosan-Containing Medical Devices, issued on March 16, 2004. In general, suitable concentrations of chlorhexidine include a range from 0.1% weight to weight (wt/wt) to 30% wt/wt. More particularly, a suitable chlorhexidine range includes from 3% wt/wt to 20% wt/wt.

Suitable base materials generally include elastomers and/or polymer materials. Examples of suitable base materials include polyurethanes, polyvinylchlorides, thermoplastics such as, for example, fluoropolymers, vinyl polymers, polyolephins, copolymers, and/or the like. The base material containing SNP, chlorhexidine, rifampin, gentian violet and/or other bioactive agents may be layered upon other bulk material to fabricate the medical device. For example, the base material having one or more bioactive constituents may be co-cxtruded with a bulk material to form layers or regions in the medical device.

In the following experiments, the use of the polymer Tecoflex®-93A resin (Lubrizol, Cleveland, OH), is specifically described. However, it is to be understood that any suitable polymer is within the scope of embodiments of this invention. Other suitable polymers include those manufactured by The Lubrizol Corp., Wickliffe, OH 44092, U.S.A., INVISTA S.à, r.l. Wichita, KS 67220, U.S.A., GLS Corp., McHenry, II 60050, U.S.A., and Colorite Polymers, Ridgefield, NJ 07657, U.S.A. In addition, chlorhexidine diacetate (George Uhe, Garfield, NJ), chlorhexidine dodecanoate (chlorhexidine laurate or chlorhexidine dilaurate), and chlorhexidine palmitate are specifically described. However, it is to be understood that any suitable chlorhexidine or salt thereof is within the scope of the embodiments of the invention. Other suitable chlorhexidine salts include chlorhexidine Myristate (chlorhexidine tetradecanoate), chlorhexidine palmitate (chlorhexidine hexadecanoate), chlorhexidine stearate (chlorhexidine octadecanoate), and various other chlorhexidines manufactured by the George Uhe Company Inc., Garfield, NJ 07026 U.S.A.

### METHODS

### EXAMPLE 1: SNP as a suitable antithrombogenic agent - effect on platelet aggregation and activation

Fresh human blood was drawn into collection tubes containing 3.8 % sodium citrate, and used within 3 hours. A fresh 25% stock of SNP (Sigma-Aldrich, St. Louis, MO) was made in 0.85% saline. 500µl of blood was mixed with 500µl warm 0.85% saline and SNP (0.05%, 0.1%, and 1%) was added and allowed to incubate at 37°C for 5 minutes with gentle stirring. Chronolog Chromolume was added and allowed to incubate for 2 minutes followed by addition of adenosine diphosphate (ADP) (10µM) to start the reaction. Platelet aggregation was measured in ohms and activation by adenosine triphosphate (ATP) release (nM) on a Chrono-Log platelet aggregometer, model 700.

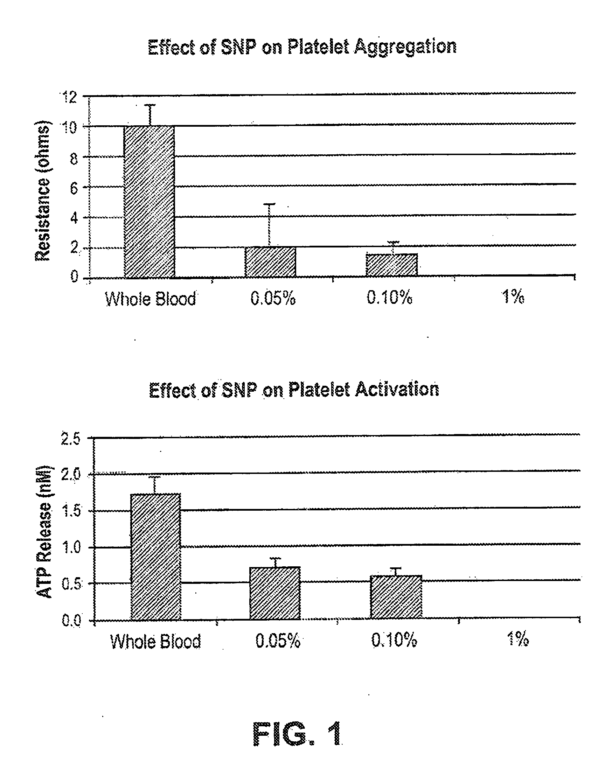

Addition of SNP at concentrations 0.1-1% in whole blood completely inhibits platelet aggregation and activation in a dose-dependent manner as shown in FIG.1 thus, showing SNP as a suitable antithrombogenic agent.

### EXAMPLE 2: Additive/synergistic effect of SNP with other antimicrobial agents against pathogenic bacteria

### Inoculum Preparation:

A few colonies of *Pseudomonas aerugionsa* ATCC 27853 were removed from secondary working cultures plated on Trypticase Soy Agar (hereinafter "TSA") with 5% sheep's blood and added to 10ml of Trypticase Soy Broth (hereinafter "TSB"). The vials were vortexed for approximately 30 seconds and incubated for 4 hours in a shaker incubator. Following incubation, they were removed and vortexed once more. The optical densities of the inoculum suspensions were read at a wavelength of 670nm. The inoculum suspensions were subsequently diluted in sterile Cation.Adjusted Mueller-Hinton Broth (hereinafter "CAMHB") to a final concentration of 1-5 x10⁶ cfu/ml.

### Antimicrobial Drug Preparation:

SNP was dissolved and diluted in sterile deionized water to get working concentrations of 1%, 0.9%, 0.8%, 0.7%, 0.6%, 0.5%, 0.4%, 0.3%, 0.2%, and 0.1% in the wells of a 96-well micro titer plate.

Chlorhexidine diacetate (hereinafter "CHA") and gentian violet (hereinafter "GV") were dissolved in sterile water. Rifampin (hereinafter "Rif") was dissolved in dimethylsulfoxide (hereinafter "DMSO"). Silver nano particles were dispersed in sterile water.

### Plate Inoculation and Incubation:

A sterile 96 well micro titer plate was used to test the following: SNP alone and SNP in combination with CHA, GV, Rif, and Silver at concentrations as stated above. All tests were performed in triplicate.

### SNP alone:

+90uL 2X CAMHB
+90uL PBS
+10uL SNP (20X)
+10uL organism diluted in 2X CAMHB
200uL total

### SNP in combination:

+90uL 2X CAMHB
+80uL PBS
+10uL SNP (20X)
+10uL Drug 2 (20X)
+10uL organism diluted in 2X CAMHB
200uL total

Once all test wells had been filled, the lid of the plate was replaced and the plate was inserted into a Ziplock™ bag and sealed to reduce evaporation. Each plate was incubated without shaking at 37°C for 18-46 hours depending on the microorganism.

### Minimum/Fractional Inhibitory Concentration Determination

After the appropriate incubation time had been met, the micro titer plate was removed from the incubator. The plate was then read on a Bio-Tek plate reader at a wavelength of 670nm with the lid off.

The results from the experiments show that SNP has either additive or synergistic effect when present in combination with other antimicrobial agents against *P*. *aeruginosa.* The most striking result is the unexpected synergy of SNP and silver. Nano-silver particles (e.g., nano-scale particles of silver) utilized in these tests have a minimum inhibitory concentration (hereinafter "MIC") of 0.2%. The inhibitory concentration of the nano-silver particles is reduced by 100 fold in response to the addition of 0.2% SNP and by 500 fold in response to the addition of 0.4% SNP as shown in Table 1. Similarly, CHA, GV and Rifampin also have lower inhibitory concentrations when used in combination with SNP as compared to when used alone against *P*. *aeruginosa* (Table 1). The inhibitory concentration of SNP alone was 0.6%.

**TABLE 1 - Compatibility of SNP in combination with antimicrobial agents against Pseudomonas aeruginosa**

| | MIC/FIC | | | |
|---|---|---|---|---|
| SNP (%) | CHP (PPM) | GV (PPM) | Nano silver (%) | Rifampin (PPM) |
| 0 | 4 | 32 | 0.2 | 16 |
| 0.2 | 4 | 4 | 0.0016 | 16 |
| 0.4 | 1 | 4 | 0.0004 | 2-4 |
| 0.6 | 0 | 0 | 0 | 0 |

### REFERENCE EXAMPLE 3: Thermostability of SNP and its synergy with silver (Ag) for enhanced NO generation

To determine if melt processing the SNP in plastics would be possible, SNP was first heated at 145°C for 10 minutes. Thereafter, the coating solutions containing unheated or heated SNP were used to prepare the NO releasing catheters. Tecothane extrusions were then dip coated in Tecoflex/THF solutions containing 0.1-1% (w/v) SNP with or without 0.1% - 1% (w/v) of nano-silver (Sigina-Aldrich, St. Louis, MO). Subsequently the dip coated extrusions were dried at room temperature for 30 minutes and cured for 2 hrs at 70 °C. The coated extrusions were characterized for NO release on a nitric oxide analyzer (Sievers® 280i manufactured by GE Analytical Instruments, Boulder, CO 80301 USA). NO released from S-nitrosoglutathione (hereinafter "GSNO"), a physiological NO donor in presence of saturated solution of cuprous chloride was used to generate a standard curve for quantification.

Tecothane extrusions coated with heated SNP (1% w/v) were able to generate 4-6nM/cm/min of nitric oxide, which is in the physiologically effective range. This indicates that SNP remained viable at high temperature opening up a range of higher temperature processes (such as melt processing) for manufacturing.

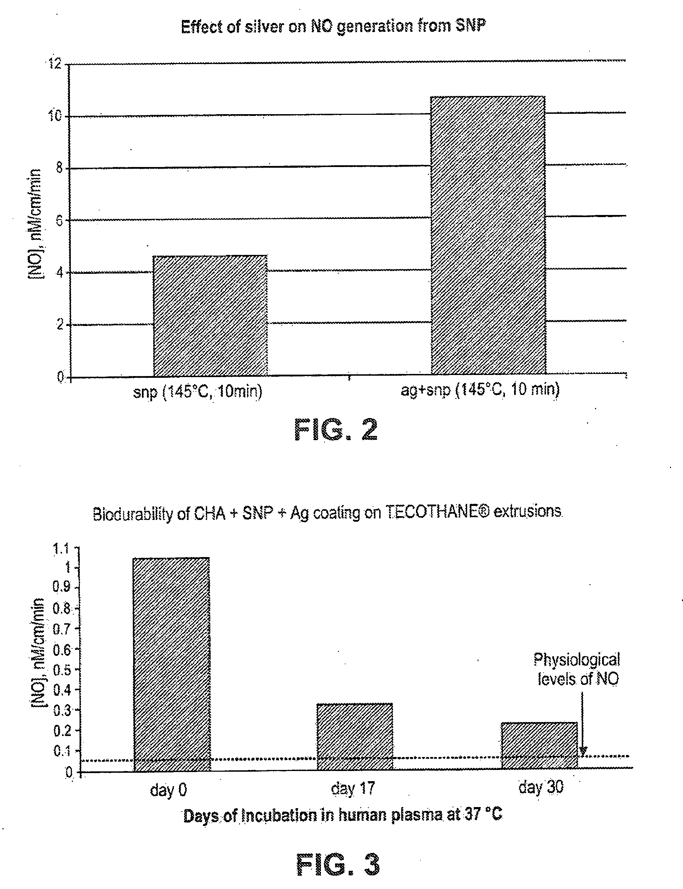

Unexpectedly, presence of silver with SNP in the coating enhanced NO release by more than two fold as shown in FIG. 2.

### EXAMPLE 4: Effect of Chlorhexidine on NO generation from SNP

Tecothane extrusions were dip coated in the SNP/Ag coating solution either with or without 3.1 % (w/w) CHA as described above in Example 3. Segments from coated extrusions were incubated in citrated human plasma at 37°C for 30 days. Plasma was replaced after every seven days of incubation. FIG. 3 shows that presence of CHA did not compromise the NO release from SNP/Ag coating and the coating remains viable even after 30 days of soaking in human plasma at 37°C.

### REFERENCE EXAMPLE 5: Effect of GV on NO generation from SNP

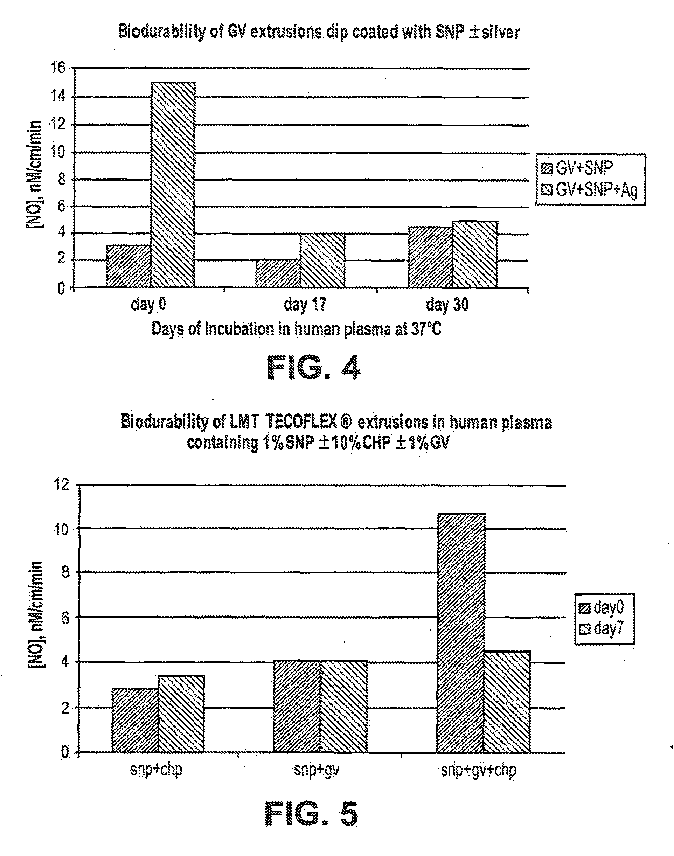

Extrusions containing 0.6% gentian violet were dip coated in the SNP solution with or without silver as described above in Example 3. Segments from coated extrusions were incubated in citrated human plasma at 37°C for 30 days. Plasma was replaced after every seven days of incubation. FIG. 4 shows presence of gentian violet does not interfere with the nitric oxide release from SNP in either presence or absence of silver.

### EXAMPLE 6: Melt processability and biodurability of the combination of SNP, GV and Chlorhexidine Palmitate

Low melt temperature, LMT-Tecothane®-93A resin (Lubrizol, Cleveland, OH) was coated with a solution of 1% w/w gentian violet (Yantai, China) and ethanol. The ethanol solvent was evaporated off in the chemical fume hood overnight and then dried at 65°C and at a pressure of 30 inches of mercury (Hg) (1.04 kilogram-force per square centimeter (kgf/cm²)) for 4 hrs prior to extrusion.

Low melting temperature Tecoflex®-93A resin, uncoated or coated with GV was mixed with or without chlorhexidine dipalmitate (hereinafter "CHP") (10% w/w) and sodium nitroprusside dihydrdate (1 % w/w) in a plastic bag. The mixture was poured in 5/8" Randcastle single screw extruder hopper (Randcastle Extrusion Systems, Inc. Cedar Grove, NJ 07009-1255 USA). The microextruder was set at 7.8 revolutions per minute (rpm) for screw speed and the barrel zone temperatures were set from 122°C (251°F) to 154°C (310°F). A size 6 French (fr) tubing was drawn from a BH25 tooling (B&H Tool Company, San Marcos, CA 92078 USA)

Extrusions with 1%SNP and either 10%CHP or 1%GV or both were incubated in.citrated human plasma at 37°C for over one week. All the three formulations after 7 days of soaking in plasma were able to generate NO at levels needed to be antithrombogenic as shown in FIG 5.

### EXAMPLE 7: Bacterial adherence on SNP/GV/CHP extrusions

Bacterial adherence on extruded tubing segments containing: 1)5% SNP;2) 1% GV + 10% CHP; and 3) 1% GV + 10% CHP + 5% SNP were compared to one another. Extrusions were performed as described in Example 6. The later was prepared targeting 10% CHP.

One centimeter long segments were cut from each of the 5% SNP, 1% GV + 10% CHP, and 1% GV + 10% CHP + 5% SNP extruded tubing and sterilized via ultra violet (UV) exposure. The segments were incubated in sterile human plasma for 0,14, and 27 days. Plasma samples were replaced with fresh plasma after every 7 days of incubation. ARROWGard blue plus (AGB⁺) catheters with chlorhexidine coating on both inside and outside surface were included as one of the negative controls.

In a 48 well micro titer plate (one per organism), wells were filled with 900µl of TSB corresponding to the predetermined number of experimental and control wells. After the wells were prepared, sterile forceps were employed to drop one experimental or control segment into a separate well. Thereafter, a suspension of each organism was prepared.

Both challenge organisms, *Staphylococcus aureus* ATCC 33591 and *Pseudomonas aerugionsa* ATCC 27853 were prepared as follows: A few colonies were removed from a secondary working culture plated on TSA with 5% sheep's blood and added to 10ml of TSB. The vials were vortexed for approximately 30 seconds and incubated for 4 hours in a shaker incubator. Following incubation, the vials were removed and vortexed once more. The bacterial absorbance of each inoculum suspension was read at an optical density of 670nm. The inoculum suspensions were then diluted to a final concentration of 1 to 5 x10⁴ colony forming units per milliliter (cfu/ml).

A volume of 100µl of the adjusted suspension was used to inoculate the sample wells (including AGB⁺ control) and growth control wells resulting in 3 logs of organisms per well. All negative control wells received a 100µl volume of TSB. The micro titer plates were then sealed with Parafilm® around the edges to minimize evaporation and incubated for 24 hours in a shaker incubator set at 37°C and 100 rpm.

Following incubation, sterile forceps were used to remove each segment and to rinse it in Phosphate Buffered Saline (hereinafter "PBS"). Each segment was rinsed individually in a separate section of a tri-divided petri dish by shaking the submerged segment back and forth approximately five times. After rinsing, the segments were placed into another 48 well plate, laid out as the original challenge plate, but contained 1ml of sterile Dey/Engley (D/E) neutralizing broth per well. The whole 48 well plate was placed into a sonication bath (250HT, VWR) and sonicated for 20 minutes at approximately 50°C. Once sonication was completed, 200µl were removed from each well and serially diluted in PBS. 10µl of each dilution was then plated onto the surface of D/E Neutralizing Agar in 12 well plates. Plates were inverted and incubated at 37°C for 24 hours. Resulting bacterial colonies were counted and log reductions in adherence were calculated between the samples and the growth controls.

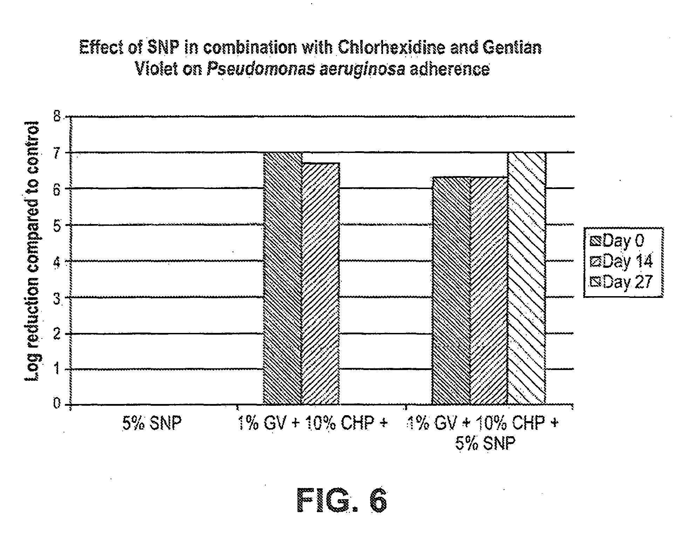

The combination of 1% GV + 10% CHP could provide protection against the gram positive bacteria, *Saphy*/*ococcus aureus* for up to 4 weeks but this combination was effective against gram negative bacteria, *Pseudomonas aeruginosa* only for less than three weeks. The addition of 5% SNP with the combination of 1% GV and 10% CHP prolonged the protection against *Pseudomonas aeruginosa* for over 4 weeks as shown in FIG. 6 (note that a 1 log reduction corresponds to a 90% reduction in the number of adherent organisms relative to an treated control; a 2 log reduction to 99%; 3 logs to 99.9% etc.).

A significant benefit of various embodiments of the invention is the ability to fabricate a SNP, GV, Ag, and/or chlorhexidine laden polymer structure in a single step. That is, the subsequent processing to introduce antibiotic agents into the extruded or molded structure that is performed during the fabrication of conventional medical devices may be omitted. In so doing, time and money may be saved.

## Claims

1. A method of fabricating a medical catheter, the method comprising:
impregnating a base material with nitroprusside;
forming the medical catheter from the base material; and
coating the medical catheter with an antimicrobial agent, wherein the antimicrobial agent comprises chlorhexidine base and/or a pharmaceutically acceptable salt thereof.

2. The method according to claim 1, further comprising:
impregnating the base material with sodium nitroprusside;

3. The method according to claim 1, further comprising:
coating the medical catheter with chlorhexidine diacetate.

4. The method according to claim 1, further comprising:
coating the medical catheter with chlorhexidine dodecanoate.

5. The method according to claim 1, further comprising:
coating the medical catheter with chlorhexidine palmitate.

6. The method according to claim 1, further comprising:
impregnating the base material with an antimicrobial dye.

7. The method according to claim 1, further including:
coating the medical catheter with an antibiotic agent.

8. The method according to claim 1, further comprising:
forming a tubular structure from the base material.

9. The method according to claim 1, wherein the base material is a polymer.

10. The method according to claim 9, wherein the base material is a polyurethane.

## Patentansprüche

1. Verfahren zur Herstellung eines medizinischen Katheters, wobei das Verfahren umfasst:
Imprägnieren eines Basismaterials mit Nitroprussid;
Ausbilden des medizinischen Katheters aus dem Basismaterial; und
Beschichten des medizinischen Katheters mit einem antimikrobiellen Mittel, wobei das antimikrobielle Mittel eine Chlorhexidin-Basis und/oder ein pharmazeutisch zulässiges Salz von diesem beinhaltet.

2. Verfahren nach Anspruch 1, weiter umfassend:
Imprägnieren des Basismaterials mit Natrium-Nitroprussid.

3. Verfahren nach Anspruch 1, weiter umfassend:
Beschichten des medizinischen Katheters mit Chlorhexidin-Diazetat.

4. Verfahren nach Anspruch 1, weiter umfassend:
Beschichten des medizinischen Katheters mit Chlorhexidin-Dodecanoat.

5. Verfahren nach Anspruch 1, weiter umfassend:
Beschichten des medizinischen Katheters mit Chlorhexidin-Palmitat.

6. Verfahren nach Anspruch 1, weiter umfassend:
Imprägnieren des Basismaterials mit einem antimikrobiellen Farbstoff.

7. Verfahren nach Anspruch 1, weiter umfassend:
Beschichten des medizinischen Katheters mit einem antibiotischen Wirkstoff.

8. Verfahren nach Anspruch 1, weiter umfassend:
Ausbilden einer rohrförmigen Struktur aus dem Basismaterial.

9. Verfahren nach Anspruch 1, wobei das Basismaterial ein Polymer ist.

10. Verfahren nach Anspruch 9, wobei das Basismaterial ein Polyurethan ist.

## Revendications

1. Procédé de fabrication d'un cathéter médical, le procédé consistant à :
imprégner un matériau de base avec du nitroprussiate ;
former le cathéter médical à partir du matériau de base ; et
recouvrir le cathéter médical avec un agent antimicrobien, l'agent antimicrobien comprenant une base de chlorhexidine et/ou un sel pharmaceutiquement acceptable de celle-ci.

2. Procédé selon la revendication 1, consistant en outre à :
imprégner le matériau de base avec du nitroprussiate de sodium.

3. Procédé selon la revendication 1, consistant en outre à :
recouvrir le cathéter médical avec du diacétate de chlorhexidine.

4. Procédé selon la revendication 1, consistant en outre à :
recouvrir le cathéter médical avec du dodécanoate de chlorhexidine.

5. Procédé selon la revendication 1, consistant en outre à :
recouvrir le cathéter médical avec du palmitate de chlorhexidine.

6. Procédé selon la revendication 1, consistant en outre à :
imprégner le matériau de base avec un colorant antimicrobien.

7. Procédé selon la revendication 1, consistant en outre à :
recouvrir le cathéter médical avec un agent antibiotique.

8. Procédé selon la revendication 1, consistant en outre à :
former une structure tubulaire à partir du matériau de base.

9. Procédé selon la revendication 1, dans lequel le matériau de base est un polymère.

10. Procédé selon la revendication 9, dans lequel le matériau de base est un polyuréthane.
